# EUROPEAN PATENT APPLICATION

(11) **EP 4 442 255 A1**
(43) Date of publication of application: **09.10.2024**
(21) Application number: 22901076.4
(22) Date of filing: 15.11.2022
(51) Int. Cl.: A61K 9/48, A23L 29/262, A61J 3/07, A61K 47/36, A61K 47/38

(54) **HARD CAPSULE, PREPARED SOLUTION FOR HARD CAPSULE, AND METHOD FOR PREPARING HARD CAPSULE**

(30) Priority: 30.11.2021 JP 2021194690
(71) Applicant: Qualicaps Co., Ltd., Nara 639-1032 (JP)
(72) Inventor: WADA, Yusuke, Yamatokoriyama-shi, Nara 639-1032 (JP); HONDA, Mamoru, Yamatokoriyama-shi, Nara 639-1032 (JP); OKOCHI, Kazuhiro, Yamatokoriyama-shi, Nara 639-1032 (JP); OSAKI, Yoshiro, Yamatokoriyama-shi, Nara 639-1032 (JP)
(74) Representative: Kraus & Lederer PartGmbB
(86) International application number: PCT/JP2022/042356
(87) International publication number: WO 2023/100643

(57) **Abstract**

The present invention addresses the problem of providing a hard capsule that can be used in a health food and has higher acid resistance.

The problem is solved by an acid-resistant hard capsule comprising a capsule shell, in which the capsule shell contains hydroxypropyl methylcellulose having a viscosity value of 15 mPa s or more and gellan gum.

## Description

### Technical Field

The present description discloses a hard capsule, a preparation solution for the hard capsule, and a method for preparing the hard capsule.

### Background Art

Currently, various formulations of capsules are used as acid-resistant hard capsules used for pharmaceutical formulations. The term "acid resistance" generally refers to properties of a formulation that make it difficult to dissolve in a stomach. On the other hand, the acid-resistant hard capsules have properties of being easily dissolved after being transferred to an intestine. For this reason, the acid-resistant hard capsules are mainly used for the purpose of protecting active pharmaceutical ingredients from gastric acid or gastric enzymes, and for the purpose of releasing the active pharmaceutical ingredients continuously while the formulation passes from a stomach to a small intestine.

Patent Literatures 1 to 4 describe hard capsules with acid resistance that use hydroxypropyl methylcellulose as a base for a capsule shell.

### Citation List

### Patent Literature

Patent Literature 1: JP2013/505928A
Patent Literature 2: JP2010/270039A
Patent Literature 3: WO2019/245031A1
Patent Literature 4: WO2019/013260A1

### Summary of Invention

### Technical Problem

However, the hard capsules described in Patent Literatures 1 to 4 all have compositions used in the pharmaceutical formulations. At least in Japanese law, there is a clear distinction between capsule components that can be used in the pharmaceutical formulations and capsule components that can be used in health foods, and it is not always possible to use the hard capsules described in Patent Literatures 1 to 4 for the health foods. In addition, hard capsules having acid resistance and usable in the health foods are currently available, but their acid resistance is not sufficient.

An object of the present invention is to provide a hard capsule having higher acid resistance and usable in the health foods.

### Solution to Problem

The present disclosure includes the following embodiments.
Item 1. An acid-resistant hard capsule comprising a capsule shell, in which the capsule shell contains hydroxypropyl methylcellulose having a viscosity value of 15 mPa·s or more and gellan gum.
Item 2. The acid-resistant hard capsule according to item 1, wherein the hydroxypropyl methylcellulose contains a first hydroxypropyl methylcellulose and a second hydroxypropyl methylcellulose whose viscosity values are different.
Item 3. The acid-resistant hard capsule according to item 1 or 2, further containing pectin.
Item 4. The acid-resistant hard capsule according to any one of items 1 to 3, wherein the dissolution rate of the acid-resistant hard capsule is 40% or less after 2 hours in a dissolution test using a solution with a pH of 1.0.
Item 5. The acid-resistant hard capsule according to any one of items 1 to 4, wherein a content of the gellan gum is in the range of 1 mass% or more to 7 mass% or less when a total mass of a solid content of the capsule shell component is set to 100 mass%.
Item 6. The acid-resistant hard capsule according to any one of items 1 to 4, wherein the content of the gellan gum is in the range of 2 mass% or more to 6 mass% or less when the total mass of the solid content of the capsule shell component is set to 100 mass%.
Item 7. The acid-resistant hard capsule according to any one of items 1 to 4, wherein the content of the gellan gum is in the range of greater than 3 mass% to 5 mass% or less when the total mass of the solid content of the capsule shell component is set to 100 mass%.
Item 8. The acid-resistant hard capsule according to any one of items 3 to 7, wherein a content of the pectin is in the range of 10 mass% or more to 50 mass% or less when the total mass of the solid content of the capsule shell component is set to 100 mass%.
Item 9. The acid-resistant hard capsule according to any one of items 3 to 7, wherein the content of the pectin is in the range of 15 mass% or more to 45 mass% or less when the total mass of the solid content of the capsule shell component is set to 100 mass%.
Item 10. The acid-resistant hard capsule according to any one of items 3 to 7, wherein the content of the pectin is in the range of 20 mass% or more to 40 mass% or less when the total mass of the solid content of the capsule shell component is set to 100 mass%.
Item 11. The acid-resistant hard capsule according to any one of items 1 to 10, containing a gelling aid in the range of 0 mass% or more to 0.003 mass% or less when the total mass of the solid content of the capsule shell component is set to 100 mass%.
Item 12. A preparation solution for preparing an acid-resistant hard capsule containing hydroxypropyl methylcellulose having a viscosity value of 15 mPa·s or more and gellan gum.
Item 13. The preparation solution according to item 12, wherein the hydroxypropyl methylcellulose contains a first hydroxypropyl methylcellulose and a second hydroxypropyl methylcellulose whose viscosity values are different.
Item 14. The preparation solution according to item 12 or 13, further containing pectin.
Item 15. The preparation solution according to any one of items 12 to 14, wherein a content of the gellan gum is in the range of 1 mass% or more to 7 mass% or less when a total mass of a solid content of a capsule shell component is set to 100 mass%.
Item 16. The preparation solution according to any one of items 12 to 14, wherein the content of the gellan gum is in the range of 2 mass% or more to 6 mass% or less when the total mass of the solid content of the capsule shell component is set to 100 mass%.
Item 17. The preparation solution according to any one of items 12 to 14, wherein the content of the gellan gum is in the range of greater than 3 mass% to 5 mass% or less when the total mass of the solid content of the capsule shell component is set to 100 mass%.
Item 18. The preparation solution according to any one of items 14 to 17, wherein a content of the pectin is in the range of 10 mass% or more to 50 mass% or less when the total mass of the solid content of the capsule shell component is set to 100 mass%.
Item 19. The preparation solution according to any one of items 14 to 17, the content of the pectin is in the range of 15 mass% or more to 45 mass% or less when the total mass of the solid content of the capsule shell component is set to 100 mass%.
Item 20. The preparation solution according to any one of items 14 to 17, wherein the content of the pectin is in the range of 20 mass% or more to 40 mass% or less when the total mass of the solid content of the capsule shell component is set to 100 mass%.
Item 21. The preparation solution according to any one of items 12 to 20, containing a gelling aid in the range of 0 mass% or more to 0.003 mass% or less when the total mass of the solid content of the capsule shell component is set to 100 mass%.
Item 22. A method for preparing an acid-resistant hard capsule comprising a step of immersing a mold pin in the preparation solution according to any one of items 12 to 21 wherein a temperature of the mold pin is lower than a temperature of the preparation solution. Advantageous Effects of Invention

It is possible to provide the hard capsule having higher acid resistance and usable for health foods. In addition, the hard capsule can be manufactured using a conventionally used hard capsule manufacturing devices.

### Description of Embodiments

### 1. Description of Terms and Materials

First, terms and materials to be used in this description, claims, and the like are described. The terms and materials regarding the present invention comply with the description in this section unless otherwise stated.

In the present invention, the term "hard capsule" refers to an empty capsule in which a content is filled into a produced capsule film. Usually, the hard capsule includes a cap portion and a body portion, and is also called a hard capsule or a two-piece capsule. The "hard capsule" in the present invention has the same or similar shape as a commercially available conventional hard capsule which is intended to be orally administered to a human or animal subject.

The "hard capsule" according to the present invention does not include a soft capsule manufactured by filling a content between two films and causing the films to adhere to each other, a seamless capsule manufactured by dropping a content together with a capsule shell solution onto a solidification liquid, and a microcapsule prepared by incorporating an active ingredient inside through precipitation or emulsification of a base material.

In addition, in the present invention, an empty hard capsule is simply referred to as "hard capsule" or "capsule", and the hard capsule filled with the content is referred to as "hard capsule formulation".

In the present invention, the term "acid-resistant hard capsule" refers to the hard capsule in which the capsule shell of a capsule main body itself has "acid resistance" properties that satisfy the following conditions.

Specifically, the term "acid resistance" refers to the properties that satisfy at least the following condition (i).
(i) A dissolution rate of a content when a test subject is immersed in an acidic solution of pH 1.0 at 37°C ± 0.5°C for 2 hours is 40% or less, preferably 30% or less. As the acidic solution, for example, 0.1 N hydrochloric acid solution can be used. The dissolution rate is obtained in accordance with a dissolution test described in the Japanese Pharmacopoeia, 17th Edition (hereinafter sometimes simply referred to as the "17th Pharmacopoeia"), except that a first fluid is changed to the acidic solution. The dissolution test may be performed in accordance with a dissolution test method specified in the 17th Pharmacopoeia (17th Pharmacopoeia, 6.10-1.2 Paddle Method (paddle rotation speed: 50 rpm), with a sinker corresponding to FIG. 6.10-2a being used). The term "acid resistance" preferably satisfies the above condition (i) as well as the following condition (ii). (ii) When the test subject is subjected to a disintegration test in accordance with the disintegration test described in the 17th Pharmacopoeia using a second fluid for dissolution test at 37°C ± 0.5°C, a disintegration time is within 2 hours, preferably within 1 hour.

The content used in the dissolution test is not limited as long as the content itself is rapidly dissolved in a test solution and can be quantified by known methods. An example is acetaminophen.

In the present description, with respect to the hydroxypropyl methylcellulose, for example, the degree of substitution with a methoxy group of the hydroxypropyl methylcellulose is preferably from 16.5 mass% to 30.0 mass%, more preferably from 19.0 mass% to 30.0 mass%, particularly preferably from 28.0 mass% to 30.0 mass%, and the degree of substitution with a hydroxypropoxy group of the hydroxypropyl methylcellulose is preferably from 4.0 mass% to 32.0 mass%, more preferably from 4.0 mass% to 12.0 mass%, particularly preferably from 7.0 mass% to 12.0 mass%.

Among these, the hydroxypropyl methylcellulose represented by the following formula is the most suitable cellulose compound because of its excellent capsule shell formability and mechanical strength under low moisture conditions. 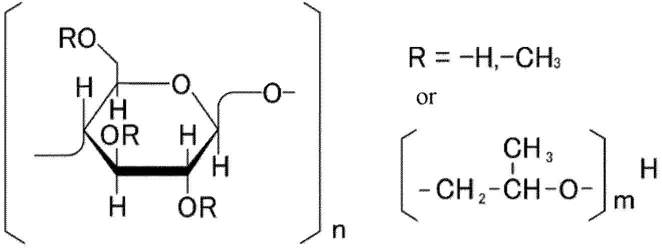 (wherein "n" and "m" represent any integers.)

The hydroxypropyl methylcellulose to be used in the present invention includes hypromellose of substitution grades (substitution types) 2910, 2906 and 2208 specified in the 17th Pharmacopoeia . Among these, the substitution types 2910 and 2906 are more preferable.

**Table 1**

| substitution type | methoxy group | | hydroxypropoxy group (%) | |
|---|---|---|---|---|
| | lower limit | upper limit | lower limit | upper limit |
| 1828 | 16.5 | 20.0 | 23.0 | 32.0 |
| 2208 | 19.0 | 24.0 | 4.0 | 12.0 |
| 2906 | 27.0 | 30.0 | 4.0 | 7.5 |
| 2910 | 28.0 | 30.0 | 7.0 | 12.0 |

In addition, the hydroxypropyl methylcellulose according to the present invention includes the hypromellose having the following molecular weight that is approved for use as a food additive in Japan.

### <Molecular Weight>

Unsubstituted structural unit: 162.14
Substituted structural unit: about 180 (degree of substitution: 1.19), about 210 (degree of substitution: 2.37)
Polymer: about 13,000 (n = about 70) to about 200,000 (n = about 1000)

Examples of commercially available hydroxypropyl methylcellulose may include for example, types 60SH and 65SH of Japanese Pharmacopoeia METOLOSE^{®}, types 90SH-100SR, 90SH-4000SR, 90SH-15000SR, and 90SH-100000SR of METROSE^{®} SR, TC-5, and types SFE, SE, and NE of METOLOSE for food additives available from Shin-Etsu Chemical Co., Ltd., TC-5 METOLOSE available from San-Ei Gen F.F.I. Inc., AnyCoat-C^{®} or AnyAddy^{®} series available from Lotte (former Samsung) Fine Chemical Co., Ltd., METHOCEL^{®} types E, F, and K series available from The Dow Chemical Company, and Benecel^{®} series available from Ashland Inc.

In the present disclosure, it is preferred to use the hydroxypropyl methylcellulose having a "viscosity value" of 15 mPa·s or more for a 2 mass% aqueous solution at 20°C. Hereinafter, this value of the viscosity may be indicated simply as "viscosity value". The "viscosity value" is measured in accordance with the section of hypromellose formulated based on International Harmonized Proposal after the Japanese Pharmacopoeia, 15th Edition. Specifically, the term "viscosity value" refers to the value of viscosity (mPa·s) at 20°C ± 0. 1°C of the 2 mass% aqueous solution of the hydroxypropyl methylcellulose. In the measurement of the "viscosity value", in the case of the "viscosity value" of less than 600 mPa·s, Method I (Ubbelohde method) in General Tests, 2.53 Viscosity Determination is used, and in the case of the "viscosity value" of 600 mPa·s or more, Method II, 2.1.2. Single cylinder-type rotational viscometer (Brookfield type viscometer) in General Tests, 2.53 Viscosity Determination is used.

In addition, as the "viscosity value", an indicated viscosity by a chemical manufacturer (sometimes referred to as "viscosity grade value") may also be adopted. Regarding the indicated viscosity and the range of the indicated viscosity, for example, in METOLOSE^{™} series manufactured by Shin-Etsu Chemical Co., Ltd., 80% to 120% of the indicated viscosity is defined in the case of the indicated viscosity of less than 600 mPa·s, and 75% to 140% of the indicated viscosity is defined in the case of the indicated viscosity of 600 mPa·s or more. Regarding the lower limit value of 15 mPa·s in the present invention, the indicated viscosity may be used directly as the "viscosity value" as long as the spirit of the present invention is not impaired. In the present disclosure, the lower limit of the preferred "viscosity value" is 15 mPa·s. The upper limit of the preferred "viscosity value" is 10,000 mPa·s.

The hydroxypropyl methylcellulose in the present invention may be a mixture of a first hydroxypropyl methylcellulose and a second hydroxypropyl methylcellulose whose viscosity values are different. Regarding the viscosity value of a mixture of two or more kinds of hydroxypropyl methylcellulose, at least one of those hydroxypropyl methylcelluloses should have a 2% viscosity value of 15 mPa·s or more in its catalog value.

The hydroxypropyl methylcellulose in a solid state is usually provided as solid fine particles with a particle size on the order of one to several hundred micrometers. Its mean particle size (mean particle diameter) is preferably in the range of 1 to 100 µm. Here, the mean particle size refers to the volume mean particle diameter (MV) of primary particles thereof, which can be determined, for example, by a commonly used laser diffraction particle size analyzer (e.g., "Microtrac Particle Size Analyzer MT3300II EX" manufactured by MicrotracBEL Corp.). Preferably, the volume fraction of the particles having a diameter of 100 µm or less is 50% or more, and even more preferably, 60% or more.

In the present disclosure, gellan gum is obtained by separating from a culture medium of a specific Gram-negative bacterium (Pseudomonas) using saccharides as a nutrient source, and its main component is a polysaccharide. The gellan gum can be classified into acylated gellan gum (native gellan gum) and deacylated gellan gum depending on the presence or absence of acylation, but both can be used in the present invention without distinction. Examples thereof may include, but are not limited to, the trade name "gellan gum" available from San-Ei Gen F.F.I. Inc. and the trade name "KELCOGEL" available from DSP Gokyo Food & Chemical Co., Ltd.

In the present disclosure, pectin is not particularly limited. Commercially available pectins include low methoxy pectin and high methoxy pectin. The low methoxy pectin is preferred. It is preferable to use the low methoxy pectin having the degree of esterification from about 15 to 50, preferably from about 20 to 45, more preferably from about 23 to 40. The degree of esterification of the pectin is according to an indicated value by a manufacturer. As the low methoxy pectin, LM-102AS-J (degree of esterification: 30), Explorer 60CS (degree of esterification: 38), LM-106AS-YA-J (degree of esterification: 23), LM-13CG-J, and others may be used. In the present disclosure, manufacturers of the pectin include, but are not limited to, San-Ei Gen F.F.I. Inc., CPKelco Inc., DSP Gokyo Food & Chemical Co., Ltd., Kyoritsu Foods Co., Ltd., and Sansho Co., Ltd.

In addition, the low methoxy pectin includes acid-treated and alkali-treated amidated pectin.

The pectin tends to have higher viscosity values and weaker acid resistance as the degree of esterification increases. If the degree of esterification is too low, the capsule shell tends not to be formed.

### 2. Hard Capsule

The first embodiment of the present disclosure relates to the hard capsule. Preferably, the hard capsule has the acid resistance.

The hard capsule comprises the capsule shell, in which the capsule shell contains the hydroxypropyl methylcellulose having the viscosity value of 15 mPa·s or more and the gellan gum. In addition, the hard capsule may contain the hydroxypropyl methylcellulose having the viscosity value of 15 mPa s or more, the gellan gum, and the pectin. In another embodiment, the hard capsule may contain the first hydroxypropyl methylcellulose and the second hydroxypropyl methylcellulose whose viscosity values are different, and the gellan gum.

When the hard capsule contains the pectin, the content of the gellan gum is in the range of 1 mass% or more to 7 mass% or less, preferably 2 mass% or more to 6 mass% or less, and more preferably greater than 3 mass% to 5 mass% or less, when a total mass of a solid content of the capsule shell component is set to 100 mass%.

The content of the pectin is in the range of 10 mass% or more to 50 mass% or less, preferably 15 mass% or more to 45 mass% or less, and more preferably 20 mass% or more to 40 mass% or less, when the total mass of the solid content of the capsule shell component is set to 100 mass%.

The content of the hydroxypropyl methylcellulose having the viscosity value of 15 mPa s or more in the hard capsule is a mass percentage (%) obtained by subtracting the mass percentage (%) of the total content of other solids from the total mass (100 mass%) of the solid content of the capsule shell component. For example, it is 35 mass% by to 99 mass%.

The hard capsule may contain a gelling aid in the range of 0 mass% or more to 0.003 mass% or less when the total mass of the solid content of the capsule shell component is set to 100 mass%. In the hard capsule of this embodiment, the gelling aid is not essential. The gelling aids may include a compound that can provide one or more of sodium, potassium, calcium, and magnesium ions in water, for example, sodium chloride, potassium chloride, calcium chloride, magnesium chloride, and magnesium sulfate. In addition, citric acid or sodium citrate may also be used as an organic acid or water-soluble salt thereof.

The capsule shell of the hard capsule may further contain a plasticizer, a surfactant (emulsifier), a binder, and the like that are acceptable pharmaceutically and as the food additives. In addition, it may also contain an agent for controlled release, an agent for assisting dissolution, a solubilizing agent, and the like to control solubility, especially, characteristics of dissolution in a neutral pH region. As the excipient acceptable as a pharmaceutical excipient, for example, the excipient listed in the Pharmaceutical Excipients Directory, 2021 edition (edited by the Japan Pharmaceutical Excipients Council, published by Yakuji Nippo, Limited) according to the above application may be used, but they are not limited thereto. Note that these additives may be classified for a plurality of applications in duplicate.

The plasticizer is not always limited to the specific substances listed in the above Pharmaceutical Excipients Directory, and are not particularly limited as long as they can be used in pharmaceutical or food compositions and can be added to the capsule shell to impart plasticity, however, suitable substances are those that generally have a molecular weight (Mw) of 100 to 20,000 and have one or a plurality of hydrophilic groups, such as a hydroxyl, ester, or amino group, in one molecule.

Examples thereof may include dioctyl adipate, polyester adipate, epoxidized soybean oil, epoxyhexahydrophthalic acid diester, kaolin, triethyl citrate, glycerin, glycerin fatty acid ester, sesame oil, dimethyl polysiloxane-silicon dioxide mixture, D-sorbitol, medium-chain fatty acid triglyceride, corn starch-derived sugar alcohol liquid, triacetin, concentrated glycerin, castor oil, phytosterol, diethyl phthalate, dioctyl phthalate, dibutyl phthalate, butyl phthalyl butyl glycolate, propylene glycol, polyoxyethylene (105) polyoxypropylene (5) glycol, polysorbate 80, macrogol 1500, macrogol 400, macrogol 4000, macrogol 600, macrogol 6000, isopropyl myristate, cotton seed oil-soybean oil mixture, glycerin monostearate, and isopropyl linoleate. The propylene glycol and polyethylene glycol are particularly suitable from the viewpoints of being excellent in compatibility and imparting high glossiness. The weight average molecular weight of the polyethylene glycol is not particularly limited, and is preferably from 200 to 35,000 from the viewpoint of imparting high glossiness. Hydroxypropyl cellulose (HPC), which is softer than MC and HPMC, may be used as the plasticizer.

Examples of the surfactant may include benzalkonium chloride, benzethonium chloride polyoxyethylene (40) monostearate (polyoxyl 40 stearate*), sorbitan sesquioleate (sorbitan sesquioleate*), polyoxyethylene (20) sorbitan monooleate (polysorbate 80*), glyceryl monostearate (glycerin monostearate*), sodium lauryl sulfate, polyoxyethylene lauryl ether (lauromacrogol*), and the like (*: notation in the Japanese Pharmacopoeia). The examples may also include sodium alkyl benzene sulfonate, sucrose fatty acid ester, polyethylene glycol monooleate, polyethylene glycol dioleate, propylene glycol fatty acid ester (such as propylene glycol monostearate and propylene glycol monocaprylate), polyoxyethylene hydrogenated castor oil, polyoxyethylene glycerin monostearate, polyoxyethylene (160) polyoxypropylene (30) glycol, polyoxyethylene nonylphenyl ether, and the like. The surfactant (or emulsifier) may also contain components that inevitably remain during emulsion polymerization of methacrylic acid copolymers and (meth)acrylic acid alkyl ester copolymers, as mentioned above.

The capsule shell of the hard capsule may further contain, for example, a lubricant, a metal sequestering agent, a colorant, a light-shielding agent, or the binder, at up to about 5 mass%. Examples of the metal sequestering agent may include ethylenediaminetetraacetic acid, acetic acid, boric acid, citric acid, gluconic acid, lactic acid, phosphoric acid, tartaric acid, or salts thereof, metaphosphate, dihydroxyethylglycine, lecithin, β-cyclodextrin, or combinations thereof.

The lubricant is not particularly limited as long as the lubricant can be used for the pharmaceutical or food composition. Examples thereof may include calcium stearate, magnesium stearate, sodium stearyl fumarate, carnauba wax, starch, sucrose fatty acid ester, light anhydrous silicic acid, talc, a hydrogenated vegetable oil, and the like.

Examples of the metal sequestering agent may include the ethylenediaminetetraacetic acid, the acetic acid, the boric acid, the citric acid, the gluconic acid, the lactic acid, the phosphoric acid, the tartaric acid, or salts thereof, the metaphosphate, the dihydroxyethylglycine, the lecithin, the β-cyclodextrin, and combinations thereof.

The colorant and the light-shielding agent are not particularly limited as long as they can be used for the pharmaceutical or food composition. Examples of the colorant may include powdered gambir tannin, turmeric extract, methylrosaniline chloride, yellow iron oxide, yellow iron sesquioxide, Opaspray K-1-24904, orange essence, brown iron oxide, carbon black, caramel, carmine, carotene liquid, β-carotene, photosensitizer 201, licorice extract, gold leaf, Sasa veitchii extract, black iron oxide, light anhydrous silicic acid, Daemonorops draco (kekketsu), zinc oxide, titanium oxide, iron sesquioxide, disazo yellow, Food Blue No. 1 and its aluminum lake, Food Blue No. 2 and its aluminum lake, Food Yellow No. 4 and its aluminum lake, Food Yellow No. 5 and its aluminum lake, Food Green No. 3 and its aluminum lake, Food Red No. 2 and its aluminum lake, Food Red No. 3 and its aluminum lake, Food Red No. 102 and its aluminum lake, Food Red No. 104 and its aluminum lake, Food Red No. 105 and its aluminum lake, Food Red No. 106 and its aluminum lake, sodium hydroxide, talc, sodium copper chlorophyllin, copper chlorophyll, powdered hull-less barley green tea extract, hull-less barley green tea extract, phenol red, sodium fluorescein, d-borneol, malachite green, octyldodecyl myristate, methylene blue, medicinal carbon, riboflavin butyrate, riboflavin, powdered green tea, manganese ammonium phosphate, riboflavin sodium phosphate, rose oil, turmeric oleoresin, chlorophyll, carminic acid, Food Red No. 40 and its aluminum lake, water-soluble annatto, sodium iron chlorophyllin, dunaliella carotene, paprika color, carrot carotene, potassium norbixin, sodium norbixin, palm oil carotene, beat red, grape skin color, black currant color, monascus color, Carthamus red color, Carthamus yellow color, marigold color, riboflavin sodium phosphate, madder color, alkanet color, aluminum, sweet potato carotene, shrimp color, krill color, orange color, cacao color, cacao carbon black, Japanese persimmon color, crab color, carob germ color, fish scale foil, silver, kusagi (Clerodendrum trichotomum) color, gardenia blue, gardenia red, gardenia yellow, kooroo color, chlorophin, kaoliang color, bone carbon black, bamboo grass color, Shea nut color, lithospermum root color, sandalwood red, vegetable carbon black, sappan wood color, spirulina color, onion color, tamarind color, corn color, tomato color, peanut color, phaffia color, pecan nut color, monascus yellow, powdered annatto, haematococcus algae color, purple sweet potato color, purple corn color, purple yam color, vegetable oil soot color, lac color, rutin, enju (Styphnolobium japonicum) extract, buckwheat whole-plant extract, logwood color, red cabbage color, red rice color, red radish color, adzuki bean color, sweet hydrangea leaf extract, sepia color, uguisukagura (Lonicera gracilipes) color, elderberry color, olive tea, cowberry color, gooseberry color, cranberry color, salmonberry color, strawberry color, dark sweet cherry color, cherry color, thimbleberry color, deberry color, pineapple juice, huckleberry color, grape juice color, black currant color, blackberry color, plum color, blueberry color, berry juice, boysenberry color, whortleberry color, mulberry color, morello cherry color, raspberry color, red currant color, lemon juice, loganberry color, powdered chlorella, cocoa, saffron color, perilla color, chicory color, laver color, hibiscus color, malt extract, paprika powder, beet red juice, carrot juice, and the like.

Examples of the light-shielding agent may include titanium oxide, iron sesquioxide, yellow iron sesquioxide, black iron oxide, Food Blue No. 1 aluminium lake, Food Blue No. 2 aluminium lake, Food Yellow No. 4 aluminium lake, Food Yellow No. 5 aluminium lake, Food Green No. 3 aluminium lake, Food Red No. 2 aluminium lake, Food Red No. 3 aluminum lake, Food Red No. 102 aluminium lake, Food Red No. 104 aluminium lake, Food Red No. 105 aluminium lake, Food Red No. 106 aluminium lake, Food Red No. 40 aluminium lake.

In order to prevent deterioration of the content due to ultraviolet rays or the like, in particular, titanium oxide may be added to the capsule shell of the hard capsule as the light-shielding agent.

It is preferable that the content of the plasticizer, surfactant (emulsifier), binder, lubricant, metal sequestering agent, colorant, light-shielding agent, and binder does not exceed 10 mass% when the total mass of the solid content of the capsule shell component is set to 100 mass%.

It is preferable that the capsule shell of the hard capsule contains 2 to 10 mass% residual moisture (water content) to maintain adequate plasticity and resistance to cracking. Usually, when the capsule after molding is dried in the range of 30 to 100°C, the residual moisture content settles to a specific saturated residual moisture level. Of course, when the drying treatment is performed at a higher temperature, the residual moisture content settles to the saturated moisture level in a shorter time. The residual moisture content depends on the environmental humidity during capsule storage, and changes almost reversibly. Specifically, the residual moisture level after sufficient drying treatment at 30 to 100°C and further storage at a constant temperature and relative humidity for several days settles to a constant saturated moisture level. In the present invention, the saturated moisture level after storage at room temperature and the relative humidity of 43% for several days is used.

The solid content of the capsule shell includes the hydroxypropyl methylcellulose, the gellan gum, and other components other than the residual moisture, such as the pectin, gelling aid, plasticizer, surfactant (emulsifier), binder, lubricant, metal sequestering agent, colorant, light-shielding agent, and binder, as appropriate.

### 3. Preparation Solution for Hard Capsule and Method for Preparing Hard Capsule

The second embodiment of the present disclosure relates to a preparation solution (also referred to simply as "preparation solution") for preparing the hard capsule described in the above section 2. The hard capsule comprises the capsule shell obtained by drying the preparation solution according to this embodiment and removing a solvent. The preparation solution for the hard capsule is also referred to as a jelly.

The preparation solution for the hard capsule contains the hydroxypropyl methylcellulose having the "viscosity value" of 15 mPa s or more, the gellan gum, and an aqueous solvent. When the capsule shell of the hard capsule contains the pectin, the preparation solution for the hard capsule contains the pectin. When the capsule shell of the hard capsule contains the gelling aid, plasticizer, surfactant (emulsifier), binder, lubricant, metal sequestering agent, colorant, light-shielding agent, or binder, the preparation solution for the hard capsule may contain these components. The hydroxypropyl methylcellulose may contain the first hydroxypropyl methylcellulose and the second hydroxypropyl methylcellulose whose viscosity values are different.

Water or a mixture of water and ethanol may be used as the aqueous solvent. The ethanol may be anhydrous ethanol. The content of the ethanol is, for example, from 10 to 20 volumes when a volume of the aqueous solvent is set to 100.

When the preparation solution for the hard capsule is prepared, the gellan gum is first added to purified water and stirred for about 1 to 10 minutes until the gellan gum is dispersed. Next, a temperature of the dispersion is raised to about 80 to 85°C, and stirred for about 10 to 120 minutes until the gellan gum is dissolved. After the gellan gum is dissolved, the hydroxypropyl methylcellulose is added while keeping a solution temperature at 80 to 85°C, and the mixture is dispersed and stirred slowly for about 60 to 180 minutes until foams disappear. Then, the solution temperature is lowered to 50 to 75°C with stirring to prepare the preparation solution for the hard capsule. When pectin is added to the capsule shell of the hard capsule, it is added after the gellan gum is dissolved but before the hydroxypropyl methylcellulose is added, and the mixture is stirred for about 10 to 120 minutes until dissolved. After dissolution, the hydroxypropyl methylcellulose is added and the mixture is dispersed and defoamed with stirring, and the temperature is lowered. When the capsule shell of the hard capsule contains the plasticizer, surfactant (emulsifier), binder, lubricant, metal sequestering agent, colorant, light-shielding agent, or binder, these components may be added at any time.

An agitation is preferably performed by rotating a propeller-shaped stirring blade at one to several hundred rpm.

After the addition of the hydroxypropyl methylcellulose and the completion of the temperature lowering step, the temperature raising step to 55 to 75°C may be performed again. Every preparation solution for the hard capsule may be subject to a step of holding at 50 to 75°C as needed to achieve an optimum viscosity after the production is completed.

The solid content of the capsule shell other than the aqueous solvent in the preparation solution for the hard capsule is preferably from 10 to 30 mass% and more preferably from 12 to 25 mass% when the preparation solution for the hard capsule is set to 100 mass%.

### 4. Method for Preparing Hard Capsule

The third embodiment of the present disclosure relates to a method for preparing the hard capsule. According to the present disclosure, the acid-resistant hard capsule may be prepared using a capsule preparation machine that prepares other hard capsules. The hard capsule is formed by an immersion method, especially, "cold pin immersion method". The "cold pin immersion method" is characterized in that a surface temperature of a mold pin during immersion is lower than the temperature of the preparation solution of the capsule.

There is no particular limitation on the method for preparing (molding) the hard capsule as long as the method comprises a step of preparing the capsule using the preparation solution for the hard capsule described in the above section 3. The hard capsule is generally formed by immersing the mold pin (a pin for molding the capsule) serving as a mold for the capsule in the preparation solution for the hard capsule, and curing and drying the capsule shell adhering to the mold pin when it is pulled up to obtain a desired capsule shape and thickness (dipping method). Specifically, the method for preparing the hard capsule comprises a step of preparing the preparation solution for the hard capsule by the method described above or preparing the preparation solution for the hard capsule through, for example, purchase thereof and a step of immersing the mold pin in the preparation solution for the hard capsule, pulling up the mold pin, inverting the mold pin upside down, and drying the solution adhering to the mold pin.

More specifically, the acid-resistant hard capsule to be used in the present invention may be produced through the following molding steps:
(1) a step of immersing the mold pin in the preparation solution for the hard capsule (immersion step);
(2) a step of pulling up the mold pin from the preparation solution for the hard capsule (immersion solution) and drying the preparation solution for the hard capsule adhering to an outer surface of the mold pin (drying step); and
(3) a step of demolding a dried capsule film (capsule shell) from the pin for molding the capsule (demolding step).

The capsule capsule shell prepared by the above method, after being cut into a predetermined length, may be provided as the acid-resistant hard capsule with the body portion and cap portion fitted or not fitted into a pair.

The thickness of the capsule shell of the hard capsule is usually in the range of 50 to 250 µm. In particular, the thickness of a side wall portion of the capsule is generally 70 to 150 µm, more preferably 80 to 120 µm for capsules currently commercially available. The acid-resistant hard capsule comes in a variety of sizes, including No. 00, No. 0, No. 1, No. 2, No. 3, No. 4, No. 5, and the like, but any size of the hard capsule may be produced in the present invention.

### 5. Acid-resistant Hard Capsule Formulation

The hard capsule of the present disclosure can be used to prepare a hard capsule formulation in which the hard capsule is filled with an active drug.

The hard capsule is suitable for filling health foods. As the health foods (including food for specified health uses, food with nutrient function claims, or food with functional claims, such as fucoidan, heme iron, polyphenols, and the like), peptides and amino acids (for example, royal jelly, ornithine, citrulline, aminolevulinic acid, black vinegar, or hydrophobic amino acids such as methionine, valine, leucine, and isoleucine), proteins (milk protein such as lactoferrin, collagen, placenta, and the like), glycoproteins, enzyme-fermented foods (such as nattokinase), coenzymes (such as coenzyme Q10), vitamins (such as beta-carotene), minerals, probiotics (such as yeast, lactic acid bacterium, bifidobacteria, etc.), plant extracts (such as crude drugs and herbs, such as turmeric extract, ginseng extract, Japanese apricot extract, ginkgo biloba extract, blueberry extract, Rubus suavissimus extract, etc.), natural organic substances such as propolis, or any combination thereof may be filled. However, it is not limited to these.

Such contents may be filled into the hard capsule using a known capsule filling machine, for example, a fully-automatic capsule filling machine (model name: LIQFIL super 80/150, manufactured by Qualicaps Co., Ltd.), a capsule filling and sealing machine (model name: LIQFIL super FS, manufactured by Qualicaps Co., Ltd.) or the like. The body portion and the cap portion of the hard capsule thus obtained are joined to each other by covering the body portion with the cap portion to fit the body portion and the cap portion with each other after the content is filled into the body portion. Then, if necessary, the filled capsule may be made tamper-proof by using appropriate techniques for sealing the seam. Typically, sealing or banding techniques may be used. Here, these techniques are well known to a person skilled in the art of capsule. As a specific example, the acid-resistant hard capsule formulation can be obtained by sealing the fitted portion by applying a sealing agent of polymer solution to the surface of the body portion and the surface of the cap portion once or a plurality of times, preferably once or twice, in the circumferential direction of the body portion and the cap portion at a constant width centered on an end edge of the cap portion. The polymer solution is not limited as long as it is a solution that forms the film, but it is preferable to use the preparation solution containing an enteric, acid-resistant, or sustained-release polymer. As the polymer solution, a diluted aqueous solution of the polymer or a solution in which the polymer is dissolved in a solvent containing a mixture of water and ethanol or a mixture of water and isopropanol may be used. When the diluted aqueous solution or the solution in which the polymer is dissolved in the solvent containing water is used, those solutions may also be used under a state in which the polymer is partially dissolved with the basic neutralizer described above. In addition, the plasticizer and the surfactant may be added. Here, the enteric polymer has a property of dissolving under neutral conditions of a small intestine (for example, in the second fluid for dissolution test described above). The acid-resistant polymer has a property of being less likely to dissolve in acidic conditions of a stomach. The sustained-release polymer has a property of being slowly dissolved in various solutions.

The polymers such as hydroxypropyl methylcellulose acetate succinate, hypromellose phthalate, methacrylic acid copolymer, ethyl acrylate-methyl methacrylate copolymer, vinyl acetate resin dispersion, ethyl cellulose, pectin, sodium alginate, potassium alginate, gellan gum, zein, shellac, hypromellose, polyvinyl alcohol, polyvinylpyrrolidone, copolypydone, carmellose sodium and the like may be dissolved in the solvent such as water, the mixture of water and ethanol, or the mixture of water and isopropanol to prepare a band seal solution. The polymer concentration in the band seal solution is not limited as long as it can function as a band seal. For example, it may be in the range of 10 mass% to 50 mass%.

In a more specific embodiment, the hydroxypropyl methylcellulose acetate succinate (HPMCAS) may be used as the enteric polymer. More specifically, the sealing solution contains 10 to 40 mass % HPMCAS-MF (manufactured by Shin-Etsu Chemical Co., Ltd.), with the remainder being the mixture of ethanol and water. Preferably, the sealing solution contains 12.5 to 35 mass% HPMCAS-MF, with the remainder being the mixture of ethanol and water. More preferably, the mixture contains 15 to 30 mass% HPMCAS-MF, with the remainder being an 80:20 mixture of ethanol and water.

For the formation of the band seal of the hard capsule formulation according to the present invention, it is preferable to use the preparative solution containing the acid-resistant polymer or the sustained-release polymer, although there is no limitation as long as the solution forms a film. Here, the preparation solution containing the acid-resistant polymer includes food materials such as pectin, alginate, and shellac, enteric bases for pharmaceuticals, and the like. The preparation solution containing the sustained-release polymer includes zein, high molecular weight water-soluble polymers, sustained-release bases for pharmaceuticals, and the like.

During capsule sealing, the preparing solution for forming the band seal may be used generally at room temperature or under heating. From the viewpoint of preventing liquid leakage of the hard capsule, it is desired to use the preparing solution for forming the band seal within a temperature range of preferably from about 23 to 45°C, more preferably from about 23 to 35°C, most preferably from about 25 to 35°C. The temperature of the preparing solution for forming the band seal may be adjusted by a method known per se, such as a panel heater or a hot-water heater. However, it is preferable to adjust the temperature by, for example, a circulating hot-water heater or a seal pan unit of the above-described integrated capsule filling and sealing machine which is remodeled into a circulating hot-water heater type, since the temperature range can be adjusted delicately.

### Examples

Hereinafter, the present invention will be described in more detail with reference to examples, but the interpretation of the present invention shall not be limited to the examples.

### I. Materials to Be Used

Materials to be used in examples and comparative examples are as described below.

### 1. Hydroxypropyl Methylcellulose

As a hydroxypropyl methylcellulose (HPMC), METROSE^{®} series, TC-5^{®} series, or SH series available from Shin-Etsu Chemical Co., Ltd., were used. Specific product names, substitution types, and "viscosity values" (indicated viscosities or viscosity grades) are as shown in Table 2.

**Table 2**

| product name | substitution type | indicated viscosity mPa·s | Description in following examples (following description of product number by manufacturer) |
|---|---|---|---|
| 60SH | 2910 | 50, 4000, 10000 | 60SH**, ** is indicated viscosity |
| 65SH | 2906 | 400 | 65SH**, ** is indicated viscosity |
| TC-5S | 2910 | 15 | |
| TC-5R | 2910 | 6 | |

### 2. Gellan Gum

A gellan gum manufactured by San-Ei Gen F.F.I. Inc. was used.

### 3. Pectin

A low methoxy pectin, LM-102AS-J or LM-106AS-YA-J manufactured by CPKelco Inc., or LM-13CG-J manufactured by Sansho Co., Ltd. was used.

### II. Preparation of Hard Capsule

In comparative examples 1 to 3, capsules were prepared under the conditions described in Table 3, following steps A to C and step D below. In examples 1, 6, and 9, capsules were prepared according to steps A to E and under the conditions shown in Tables 3 and 4. In examples 3, 4, 7, 8, and 10 to 13, capsules were prepared according to steps A to C and E and under the conditions shown in Tables 3 and 4. Example 5 is described below. In the comparative examples and examples, ingredients were weighed according to preparation quantities shown in Tables 5 and 6.

Details of the steps A to E are described below. The steps A to D are steps of preparing a preparation solution for the capsule, and the step E is a step of preparing a capsule capsule shell.

The step A is a step of dispersing a gellan gum and dissolving the gellan gum and a pectin. A mixture containing the gellan gum was stirred until the gellan gum was dispersed for about 1 to 10 minutes, and then stirred until the gellan gum dissolved for about 10 to 120 minutes after the temperature was raised. The pectin was then added and the mixture was stirred for about 10 to 120 minutes until dissolved.

In a formulation without addition of the pectin, only the gellan gum was dissolved.

The step B is a step of dispersing and defoaming the HPMC. A mixture of containing the HPMC is slowly stirred for about 1 to 3 hours after the dispersion until foams disappear.

The step C is a step of lowering the temperature. The temperature was lowered to an optimum temperature over a period of about 1 to 3 hours and the mixture was stirred using a three-one motor. In the examples 1, 6, and 9, the temperature in the step C was set at 52°C for the purpose of adjusting a viscosity of the preparation solution for the capsule. In the comparative examples 1 to 3, the examples 3, 4, 7, 8, and 10 to 13, the temperature of the step C was set at 60°C or 55°C as shown in Tables 3 and 4. For capsule preparation in the examples 1, 6, and 9, the step C was followed by the step D. When preparing the comparative examples 1 to 3, examples 3, 4, 7, 8, and 10 to 13, after the step C, the step E was carried out without carrying out the step D. The step D is a step of raising the temperature. The temperature was raised to an optimum temperature over a period of about 1 to 3 hours and the mixture was stirred using the three-one motor. In the examples 1, 6, and 9, since the temperature of the preparation solution for the capsule in the step C was low, when preparing the capsules of these examples, the step D was carried out before proceeding to the step E, so that the viscosity of the preparation solution for the capsule was adjusted to be suitable for dipping by raising the temperature of the preparation solution for the capsule.

The step E is a step of holding and dipping. After the preparation solution for the capsule poured into a dip pan was held at the optimum temperature for dipping, a mold pin (size No. 2), which had been left at room temperature (about 25°C), was immersed in the solution for a few seconds and then pulled up into the air to dry. The mold pin to which the preparation solution for the capsule adhered was inverted upside down and dried at room ambient temperature for 10 hours or more. The optimum temperature in the step E is intended to be a temperature suitable for preparing the capsules that conform to the standard when the mold pin is immersed in the preparation solution for the capsule in the comparative examples and examples.

In the comparative example 4 and the examples 14 to 19, the step A was carried out as follows.

The gellan gum was stirred in the purified water at 65°C for about 1 to 10 minutes until dispersed. After dispersing the gellan gum, the pectin was added and the mixture was stirred for about 10 to 120 minutes until dispersed. The dispersion was then heated to 85°C and stirred until the gellan gum and the pectin dissolved. The steps B to E were carried out in the same manner as described above under the conditions shown in Table 14.

The capsule shell prepared as described above was cut into predetermined lengths to prepare hard capsule formulations for performance testing. The hard capsule formulations were prepared with and without applying a band seal to a fitted portion between a cap portion and a body portion.

The band seal was prepared and applied by the following method.

Hydroxypropyl methylcellulose acetate succinate (HPMCAS), purified water, and ethanol were mixed to final concentrations of 20 mass%, 16 mass%, and 64 mass%, respectively, and the HPMCAS was dissolved therein to prepare a band seal solution. The band seal was applied by applying the band seal solution in a band shape with a width of about 5 mm in the circumferential direction of the fitted portion so as to cover the fitted portion between the cap portion and the body portion of the capsule, and drying at room temperature.

An example of the preparation of the example 5 is described below.

Gellan gum (6.75 g) was added to purified water and stirred with a three-one motor for about 5 minutes until dispersed, then the temperature of the mixture was raised to 85°C and stirred for about 10 minutes until the gellan gum was dissolved.

While maintaining the temperature of the solution at 85°C, pectin LM-102AS-J (45 g) was added and stirred with a three-one motor for about 60 minutes until dissolved (Step A).

While maintaining the temperature of the jelly at 85°C, 60SH-10000 (128.25 g) and 60SH-50 (45 g) were added and dispersed, and the mixture was stirred slowly for about 3 hours until foams disappear (Step B).

The temperature was then lowered to 52°C with continued stirring, and the mixture was stirred for about 1 hour (Step C).

After lowering the temperature, the temperature was raised again to 65°C and stirred for about 1 hour (Step D).

A preparation solution for a capsule prepared above was poured into a dip pan and hard capsules were prepared by cold pin immersion method. A mold pin (size No. 2) that had been left at room temperature (about 25°C) was immersed for a few seconds in the preparation solution for the capsule maintained at 58 to 63°C in the dip pan, and then pulled up into the air. The mold pin to which the preparation solution for the capsule adhered was inverted upside down and dried at room ambient temperature for 10 hours or more (Step E).

A capsule shell prepared as described above was cut into predetermined lengths to prepare hard capsule formulations for performance testing. The hard capsule formulations were prepared with and without applying a band seal to a fitted portion between a cap portion and a body portion.

The band seal was prepared and applied in the same manner as in the other preparations.

### Table 3

**Table 3**

| | | comparative example 1 | comparative example 2 | comparative example 3 | example 1 | example 2 | example 3 | example 4 | example 5 |
|---|---|---|---|---|---|---|---|---|---|
| step A | temperature raising | 85°C | 85°C | 85°C | 85°C | 80°C | 85°C | 80°C | 85°C |
| step B | dispersing and defoaming | 85°C | 85°C | 85°C | 85°C | 80°C | 85°C | 80°C | 85°C |
| step C | temperature lowering | 60°C | 55°C | 55°C | 52°C | 55°C | 55°C | 55°C | 52°C |
| step D | temperature raising | - | - | - | 65°C | - | - | - | 65°C |
| step E | holding and dipping | 60 - 65°C | 53 - 58°C | 58 - 63°C | 60 - 65°C | 65 - 70°C | 62 - 67C | 65 - 70°C | 58 - 63°C |

### Table 4

**Table 4**

| | | example 6 | example 7 | example 8 | example 9 | example 10 | example 11 | example 12 | example 13 |
|---|---|---|---|---|---|---|---|---|---|
| step A | temperature raising | 85°C | 85°C | 85°C | 85°C | 85°C | 85°C | 85°C | 85°C |
| step B | dispersing and defoaming | 85°C | 85°C | 85°C | 85°C | 85°C | 85°C | 85°C | 85°C |
| step C | temperature lowering | 52°C | 55°C | 55°C | 52°C | 55°C | 55°C | 60°C | 60°C |
| step D | temperature raising | 65°C | - | - | 65°C | - | - | - | - |
| step E | holding and dipping | 58 - 63°C | 63 - 68°C | 57 - 62°C | 55 - 60°C | 63 - 68°C | 60 - 65°C | 57 - 62°C | 57 - 62°C |

### Table 5

**Table 5**

| component | 2% viscosity (mPa · S) | product name | comparativ e example 1 | comparativ e example 2 | comparativ e example 3 | example 1 | example 2 | example 3 | example 4 | example 5 |
|---|---|---|---|---|---|---|---|---|---|---|
| HPMC 2910 | 6 | TC5R | - | 78.69 | - | - | - | - | - | - |
| HPMC 2910 | 15 | TC-5S | - | - | - | - | 36.40 | 54.60 | - | - |
| HPMC 2910 | 50 | 60SH-50 | - | - | - | - | - | - | - | 45.00 |
| HPMC 2906 | 400 | 65SH-400 | - | - | - | - | - | - | - | - |
| HPMC 2910 | 4000 | 60SH-4000 | - | - | - | - | - | - | - | - |
| HPMC 2910 | 10000 | 60SH-10000 | 102.20 | - | 81.76 | 203.70 | 51.87 | 33.67 | 78.69 | 128.25 |
| pectin | | LM-102AS-J | - | 20.44 | 20.44 | - | - | - | 20.44 | 45.00 |
| gellan gum | | gellan gum | - | 3.07 | - | 6.30 | 2.73 | 2.73 | 3.07 | 6.75 |
| purified water | | purified water | 627.8 | 627.8 | 627.8 | 1190 | 559 | 559 | 627.8 | 1275 |
| solid content in total prepared solution | | | 14% | 14% | 14% | 15% | 14% | 14% | 14% | 15% |

### Table 6

**Table 6**

| component | 2% viscosity (mPa · S) | product name | example 6 | example 7 | example 8 | example 9 | example 10 | example 11 | example 12 | example 13 |
|---|---|---|---|---|---|---|---|---|---|---|
| HPMC 2910 | 6 | TC5R | - | - | - | - | - | - | - | - |
| HPMC 2910 | 15 | TC-5S | - | - | 78.69 | - | - | - | - | - |
| HPMC 2910 | 50 | 60SH-50 | - | - | - | 161.70 | - | - | - | - |
| HPMC 2906 | 400 | 65SH-400 | - | - | - | - | 78.69 | - | - | - |
| HPMC 2910 | 4000 | 60SH-4000 | - | - | - | - | - | 78.69 | - | - |
| HPMC 2910 | 10000 | 60SH-10000 | 182.70 | 51.87 | - | - | - | - | 82.95 | 78.75 |
| pectin | | LM-102AS-J | 21.00 | 36.40 | 20.44 | 42.00 | 20.44 | 20.44 | 21.00 | 21.00 |
| gellan gum | | gellan gum | 6.30 | 2.73 | 3.07 | 6.30 | 3.07 | 3.07 | 1.05 | 5.25 |
| purified water | | purified water | 1190 | 559 | 627.8 | 1190 | 627.8 | 627.8 | 645 | 645 |
| solid content in total prepared solution | | | 15% | 14% | 14% | 15% | 14% | 14% | 14% | 14% |

### III. Dissolution Test

The solubility (dissolution characteristics) of a capsule itself prepared in the above section II was evaluated by evaluating dissolution of fast-dissolving acetaminophen. 40 mg of acetaminophen, 140 mg of lactose, and 20 mg of sodium starch glycolate were filled into one capsule, and the resulting hard capsule formulation was tested in accordance with a dissolution test method specified in the Japanese Pharmacopoeia (17th Pharmacopoeia, 6.10-1.2 Paddle Method (paddle rotation speed: 50 rpm), with a sinker corresponding to FIG. 6.10-2a being used), to thereby measure a change over time of a dissolution rate of the acetaminophen. A bath-type dissolution tester Model 2100 manufactured by Distek Inc. was used for the dissolution test. The absorbance at 244 nm when the same volume of acetaminophen was separately dissolved in an entire amount in the solution in the dissolution tester bath was set to 100%, and the dissolution rate was determined based on the absorbance at 244 nm in the solution in the dissolution tester bath that increased in association with dissolution of the acetaminophen from the capsule. The dissolution test was performed using 0.1N hydrochloric acid (pH 1.0) as a dissolution test solution in a volume of 1000 ml. The number of samples was set to 3 to 6, and the average value was used as the dissolution rate.

### IV. Disintegration Test

Disintegration test was performed in accordance with section "6.09 Disintegration test method" described in the Japanese Pharmacopoeia, 17th Edition. This disintegration test was performed using an apparatus and a disk that meet the conditions described in the section "6.09 Disintegration test method". An overview of the equipment and the disk is described below.

### 1. Test Apparatus

(i) The apparatus used for the disintegration test consists of a basket-rack assembly, a 1000-mL low-form beaker with a height of 138 to 160 mm and an inside diameter of an immersion part of 97 to 115 mm, a thermostatic bath with temperature controllable at 37 ± 2°C, and a device for raising and lowering the basket at a constant frequency rate between 29 and 32 cycles per minute through a distance of not less than 53 mm and not more than 57 mm. The volume of an immersion fluid in the beaker was such that when the basket was at the highest point of the upward stroke, a wire mesh of the basket remains at least 15 mm below the surface of the fluid and when the basket was at its lowest point of the downward stroke, the wire mesh of the basket was at least 25 mm above the bottom of the beaker, so that the basket-rack assembly was not completely submerged. The time required for the upward stroke of the device is equal to the time required for the downward stroke of the device, and the change in stroke direction was made to be smooth rather than abrupt. The basket-rack assembly moved vertically along its axis, and the axis was not moved or shifted horizontally.
(ii) The basket-rack assembly consists of six open-ended transparent glass tubes, each 77.5 ± 2.5 mm in length, 20.7 to 23 mm in inside diameter, and 1.0 to 2.8 mm in wall thickness, and two plastic plates for holding the tubes in a vertical position, each 88 to 92 mm in diameter and 5 to 8.5 mm in thickness, having six holes, each 22 to 26 mm in diameter, equidistant from the center of the plate and equally spaced from one another. Attached to the under surface of the lower plastic plate is a woven stainless steel wire cloth, which has a plain square weave with 1.8 to 2.2 mm apertures and with a wire diameter of 0.57 to 0.66 mm. The parts of the basket-rack assembly are assembled and rigidly held by means of three bolts passing through the two plastic plates. The basket-rack assembly conforms to the dimensions found in FIG. 6.09-1 described in the Japanese Pharmacopoeia, 17th Edition. The basket-rack assembly was suspended from the device so that it could move up and down along its central axis.
(iii) One disk was placed in each glass tube. The disk is cylindrical in shape, 9.5 ± 0.15 mm in height and 20.7 ± 0.15 mm in diameter, and is made of transparent plastic with a specific gravity of 1.18 to 1.20. Five parallel holes of 2 ± 0.1 mm in diameter extend between the upper and lower sides of the disk. One of the holes is centered on the disk, while the other four are equally spaced at a distance of 6 ± 0.2 mm from the center of the disk. Four identical trapezoidal-shaped planes are cut into the side wall of the disk, nearly perpendicular to the ends of the disk. The trapezoid is symmetrical; its parallel sides coincide with the ends of the disk and are parallel to an imaginary line connecting the centers of two adjacent holes 6 mm from the cylindrical axis. The parallel side of the trapezoid on the bottom of the disk has a length of 1.6 ± 0.1 mm, and its bottom edges lie at a depth of 1.5 to 1.8 mm from the circumference of the disk, while the parallel side of the trapezoid on the top of the disk has a length of 9.4 ± 0.2 mm, and its center lies at a depth of 2.6 ± 0.1 mm from the circumference of the disk. The disk conforms to dimensions found in FIG. 6.09-1, and all surfaces of the disk are smooth.

### 2. Procedure

The sample capsules were placed in each of the six glass tubes of the basket with one sample in each tube, and the apparatus was operated at 37 ± 2°C using a second fluid for dissolution test as the immersion fluid. When appropriate, lift the basket from the fluid, and observe the disintegration of the sample. Complete disintegration of the sample is defined as a state in which any residue of the capsule filling has not been observed in the glass tube, and the disintegration time of six samples was measured.

As in the dissolution test, each sample capsule was filled with 40 mg of acetaminophen, 140 mg of lactose, and 20 mg of sodium starch glycolate, and the resulting hard capsule formulation was used for the test. All capsules used in the test were not applied the band seal.

### V. Results

Tables 7 to 12 and 15 show the formulations and dissolution rates of the capsules of the comparative examples 1 to 4 and the examples 1 to 19 prepared. In Tables 7 to 12 and 15, the content of each component is shown in terms of mass% of each component when the solid content of the capsule shell component is set to 100 mass%. When immersed in hydrochloric acid solution of pH 1.0 for 2 hours, the dissolution rate of the comparative example 1 and the comparative example 2 was 99.4% and 71.5%, respectively, while the dissolution rates of the examples 1 to 19 were 40% or less indicating an improvement in acid resistance. The results of the examples 2 and 3 which are a combination of two types of the HPMC, whose viscosity values are different, and the gellan gum, and the examples 4 to 19 which are a combination of the HPMC having a viscosity values of 15 mPa·s or more, the gellan gum, and the pectin, also showed that the dissolution rates of these hard capsules were 40% or less when immersed in the hydrochloric acid solution of pH 1.0 for 2 hours, indicating an improvement in acid resistance.

Table 13 shows the results of the disintegration test. All capsules tested in the disintegration test using the second fluid for dissolution test disintegrated within one hour.

### Table 7

**Table 7**

| component | 2% viscosity (mPa · S) | product name | comparative example 1 | example 1 |
|---|---|---|---|---|
| HPMC 2910 | 10000 | 60SH-10000 | 100 | 97 |
| pectin | | LM-102AS-J | - | - |
| gellan gum | | gellan gum | - | 3 |
| total | | | 100 | 100 |
| dissolution rate of capsules at pH 1.0 for 2 hours (%) | | with band seal | 99.4(1.1) | 30.5(12.4) |
| S.D is shown in parentheses | | without band seal | 97.1(2.1) | 31.5(7.1) |

### Table 8

**Table 8**

| component | 2% viscosity (mPa · S) | product name | example 1 | example 2 | example 3 |
|---|---|---|---|---|---|
| HPMC 2910 | 15 | TC-5S | - | 40 | 60 |
| HPMC 2910 | 50 | 60SH-50 | - | - | - |
| HPMC 2910 | 10000 | 60SH-10000 | 97 | 57 | 37 |
| pectin | | LM-102AS-J | - | - | - |
| gellan gum | | gellan gum | 3 | 3 | 3 |
| total | | | 100 | 100 | 100 |
| dissolution rate of capsules at pH 1.0 for 2 hours (%) | | with band seal | 30.5(12.4) | 21.6( 1.0) | 26.5( 2.5) |
| S.D is shown in parentheses | | without band seal | 31.5(7.1) | 28.9( 1.9) | - |

### Table 9

**Table 9**

| component | 2% viscosity (mPa · S) | product name | example 4 | example 5 |
|---|---|---|---|---|
| HPMC 2910 | 50 | 60SH-50 | | 20 |
| HPMC 2910 | 10000 | 60SH-10000 | 77 | 57 |
| pectin | | LM-102AS-J | 20 | 20 |
| gellan gum | | gellan gum | 3 | 3 |
| total | | | 100 | 100 |
| dissolution rate of capsules at pH 1.0 for 2 hours (%) | | with band seal | 17.1(1.8) | 21.0(1.6) |
| S.D is shown in parentheses | | without band seal | 25.4(2.8) | 25.5(2.1) |

### Table 10

**Table 10**

| component | 2% viscosity (mPa · S) | product name | example 1 | example 6 | example 4 | example 7 |
|---|---|---|---|---|---|---|
| HPMC 2910 | 10000 | 60SH-10000 | 97 | 87 | 77 | 57 |
| pectin | | LM-102AS-J | - | 10 | 20 | 40 |
| gellan gum | | gellan gum | 3 | 3 | 3 | 3 |
| total | | | 100 | 100 | 100 | 100 |
| dissolution rate of capsules at pH 1.0 for 2 hours (%) | | with band seal | 30.5(12.4) | 27.3(10.1) | 17.1(1.8) | 29.3(1.6) |
| S.D is shown in parentheses | | without band seal | 31.5(7.1) | 32.3(7.8) | 25.4(2.8) | 36.7(6.3) |

### Table 11

**Table 11**

| component | 2% viscosity (mPa · S) | product name | comparative example 2 | example 8 | example 9 | example 10 | example 11 | example 4 |
|---|---|---|---|---|---|---|---|---|
| HPMC 2910 | 6 | TC5R | 77 | - | - | - | - | - |
| HPMC 2910 | 15 | TC-5S | - | 77 | - | - | - | - |
| HPMC 2910 | 50 | 60SH-50 | - | - | 77 | - | - | - |
| HPMC 2906 | 400 | 65SH-400 | - | - | - | 77 | - | - |
| HPMC 2910 | 4000 | 60SH-4000 | - | - | - | - | 77 | - |
| HPMC 2910 | 10000 | 60SH-10000 | - | - | - | - | - | 77 |
| pectin | | LM-102AS-J | 20 | 20 | 20 | 20 | 20 | 20 |
| gellan gum | | gellan gum | 3 | 3 | 3 | 3 | 3 | 3 |
| total | | | 100 | 100 | 100 | 100 | 100 | 100 |
| dissolution rate of capsules at pH 1.0 for 2 hours (%) S.D is shown in parentheses | | with band seal | 71.5(12.3) | 35.3(3.4) | 35.1(3.9) | 21.8(1.3) | 22.8(3.1) | 17.1(1.8) |
| | | without band seal | 70.1(1.2) | - | - | - | - | 25.4(2.8) |

### Table 12

**Table 12**

| component | 2% viscosity (mPa · S) | product name | comparative example 3 | example 12 | example 4 | example 13 |
|---|---|---|---|---|---|---|
| HPMC 2910 | 10000 | 60SH-10000 | 80 | 79 | 77 | 75 |
| pectin | | LM-102AS-J | 20 | 20 | 20 | 20 |
| gellan gum | | gellan gum | 0 | 1 | 3 | 5 |
| total | | | 100 | 100 | 100 | 100 |
| dissolution rate of capsules at pH 1.0 for 2 hours (%) S.D is shown in parentheses | | with band seal | 49.2(3.3) | 19.8(0.5) | 17.1(1.8) | 20.5(1.5) |
| | | without band seal | 54.1(9.3) | 29.5(9.2) | 25.4(2.8) | 28.2(1.9) |

### Table 13

**Table 13**

| component | 2% viscosity (mPa · S) | product name | example 1 | example 4 | example 6 | example 9 |
|---|---|---|---|---|---|---|
| HPMC 2910 | 50 | 60SH-50 | - | - | - | 77 |
| HPMC 2910 | 10000 | 60SH-10000 | 97 | 77 | 87 | - |
| pectin | | LM-102AS-J | - | 20 | 10 | 20 |
| gellan gum | | gellan gum | 3 | 3 | 3 | 3 |
| total | | | 100 | 100 | 100 | 100 |
| disintegration time in second fluid for dissolution test (N6) | | | within 50 minutes | within 45 minutes | within 50 minutes | within 30 minutes |

### ]Table 14

**Table 14**

| | | comparative example 4 | example 14 | example 15 | example 16 | example 17 | example 18 | example 19 |
|---|---|---|---|---|---|---|---|---|
| step A | temperature raising | 85°C | 85°C | 85°C | 85°C | 85°C | 85°C | 85°C |
| step B | dispersing and defoaming | 85°C | 85°C | 85°C | 85°C | 85°C | 85°C | 85°C |
| step C | temperature lowering | 52°C | 52°C | 52°C | 52°C | 52°C | 52°C | 52°C |
| step D | temperature raising | 55°C | 55°C | 60°C | 60°C | 60°C | 60°C | 60°C |
| step E | holding and dipping | 54∼63°C | 54 - 63°C | 56°C | 56°C | 58°C | 54 - 63°C | 54 - 63°C |

### Table 15

**Table 15**

| component | 2% viscosity (mPa · S) | product name | comparative example 4 | example 14 | example 15 | example 16 | example 17 | example 18 | example 19 |
|---|---|---|---|---|---|---|---|---|---|
| HPMC 2906 | 400 | 65SH-400 | - | - | - | - | 76.5 | - | - |
| HPMC 2910 | 4000 | 60SH-4000 | - | - | - | 76.5 | - | - | - |
| HPMC 2910 | 100000 | 60SH-10000 | 79.5 | 79 | 76.5 | - | - | 76.5 | 76.5 |
| pectin | | LM-102AS-J | 20 | 20 | 20 | 20 | 20 | - | - |
| | | LM-106AS-YA-J | - | - | - | - | - | 20 | - |
| | | LM-13CG-J | - | - | - | - | - | - | 20 |
| gellan gum | | gellan gum | 0.5 | 1 | 3.5 | 3.5 | 3.5 | 3.5 | 3.5 |
| total | | | 100 | 100 | 100 | 100 | 100 | 100 | 100 |
| dissolution rate of capsules at pH 1.0 for 2 hours (%) S.D is shown in parentheses | | with band seal | 45.8(21.5) | 26.8(6.6) | 18.1(1.5) | 20.8(2.0) | 31.2(8.5) | 19.4(3.7) | 27.8(6.6) |
| | | without band seal | 49.8(14.4) | 37.1(5.6) | 34.3(4.4) | 36.5(6.4) | - | 32.4(2.3) | 29.9(3.0) |

## Claims

1. An acid-resistant hard capsule comprising a capsule shell, in which the capsule shell contains hydroxypropyl methylcellulose having a viscosity value of 15 mPa s or more and gellan gum.

2. The acid-resistant hard capsule according to claim 1, wherein the hydroxypropyl methylcellulose contains a first hydroxypropyl methylcellulose and a second hydroxypropyl methylcellulose whose viscosity values are different.

3. The acid-resistant hard capsule according to claim 1, further containing pectin.

4. The acid-resistant hard capsule according to claim 1, wherein the dissolution rate of the acid-resistant hard capsule is 40% or less after 2 hours in a dissolution test using a solution with a pH of 1.0.

5. The acid-resistant hard capsule according to claim 1, wherein the content of the gellan gum is in the range of 1 mass% or more to 7 mass% or less when the total mass of a solid content of the capsule shell component is set to 100 mass%.

6. The acid-resistant hard capsule according to claim 1, wherein the content of the gellan gum is in the range of 2 mass% or more to 6 mass% or less when the total mass of the solid content of the capsule shell component is set to 100 mass%.

7. The acid-resistant hard capsule according to claim 1, wherein the content of the gellan gum is in the range of greater than 3 mass% to 5 mass% or less when the total mass of the solid content of the capsule shell component is set to 100 mass%.

8. The acid-resistant hard capsule according to claim 3, wherein the content of the pectin is in the range of 10 mass% or more to 50 mass% or less when the total mass of the solid content of the capsule shell component is set to 100 mass%.

9. The acid-resistant hard capsule according to claim 3, wherein the content of the pectin is in the range of 15 mass% or more to 45 mass% or less when the total mass of the solid content of the capsule shell component is set to 100 mass%.

10. The acid-resistant hard capsule according to claim 3, wherein the content of the pectin is in the range of 20 mass% or more to 40 mass% or less when the total mass of the solid content of the capsule shell component is set to 100 mass%.

11. The acid-resistant hard capsule according to claim 1, containing a gelling aid in the range of 0 mass% or more to 0.003 mass% or less when the total mass of the solid content of the capsule shell component is set to 100 mass%.

12. A preparation solution for preparing an acid-resistant hard capsule containing hydroxypropyl methylcellulose having a viscosity value of 15 mPa·s or more and gellan gum.

13. The preparation solution according to claim 12, wherein the hydroxypropyl methylcellulose contains a first hydroxypropyl methylcellulose and a second hydroxypropyl methylcellulose whose viscosity values are different.

14. The preparation solution according to claim 12, further containing pectin.

15. The preparation solution according to claim 12, wherein the content of the gellan gum is in the range of 1 mass% or more to 7 mass% or less when the total mass of the solid content of the capsule shell component is set to 100 mass%.

16. The preparation solution according to claim 12, wherein the content of the gellan gum is in the range of 2 mass% or more to 6 mass% or less when the total mass of the solid content of the capsule shell component is set to 100 mass%.

17. The preparation solution according to claim 12, wherein the content of the gellan gum is in the range of greater than 3 mass% to 5 mass% or less when the total mass of the solid content of the capsule shell component is set to 100 mass%.

18. The preparation solution according to claim 14, wherein the content of the pectin is in the range of 10 mass% or more to 50 mass% or less when the total mass of the solid content of the capsule shell component is set to 100 mass%.

19. The preparation solution according to claim 14, wherein in the range of 15 mass% or more to 45 mass% or less when the total mass of the solid content of the capsule shell component is set to 100 mass%.

20. The preparation solution according to claim 14, wherein the content of the pectin is in the range of 20 mass% or more to 40 mass% or less when the total mass of the solid content of the capsule shell component is set to 100 mass%.

21. The preparation solution according to claim 12 containing a gelling aid in the range of 0 mass% or more to 0.003 mass% or less when the total mass of the solid content of the capsule shell component is set to 100 mass%.

22. A method for preparing an acid-resistant hard capsule comprising a step of immersing a mold pin in the preparation solution according to claim 12 wherein the temperature of the mold pin is lower than the temperature of the preparation solution.
